# EUROPEAN PATENT APPLICATION

(11) **EP 3 992 595 A1**
(43) Date of publication of application: **04.05.2022**
(21) Application number: 20833571.1
(22) Date of filing: 15.05.2020
(51) Int. Cl.: G01J 1/02, B23K 26/57, G01N 21/59, H03K 17/78, B29C 65/16, H01S 3/00

(54) **PHOTOELECTRIC SENSOR, METHOD FOR MEASURING RESIN TRANSMITTANCE IN LASER RESIN WELDING, LASER RESIN WELDING METHOD, AND LASER MACHINING DEVICE**

(30) Priority: 26.06.2019 JP 2019118973
(71) Applicant: Panasonic Intellectual Property Management Co., Ltd., Osaka-shi, Osaka 540-6207 (JP)
(72) Inventor: OGAWA Yoshiki, Osaka 540-6207 (JP)
(74) Representative: SSM Sandmair
(86) International application number: PCT/JP2020/019527
(87) International publication number: WO 2020/261803

(57) **Abstract**

A photoelectric sensor of the present disclosure includes: a placement table (33a) configured to allow a workpiece (W1) to be placed thereon; light projecting means (4) including a laser diode (41) and a converging lens (45); and a photodiode (52) configured to receive a laser beam (LB) passing through a detection area (DA) at a position located on the same plane as the placement table (33a). An optical axis of the laser beam (LB) projected from the converging lens (45) is set such that the laser beam (LB) is incident in a direction perpendicular to an incident surface of the photodiode (52) and focused on the incident surface of the photodiode (52).

## Description

### TECHNICAL FIELD

The present disclosure relates to a photoelectric sensor including light projecting means and light receiving means, a method for measuring resin transmittance in laser resin welding, a laser resin welding method, and a laser machining device, and more particularly, to a photoelectric sensor suitable for determining resin transmittance for laser welding of a laser transmissive resin when resins are overlapped and welded by a laser, a method for measuring resin transmittance in laser resin welding using the photoelectric sensor, a laser resin welding method based on the measured resin transmittance, and a laser machining device used in the laser resin welding method.

### BACKGROUND ART

In order to overlap resins and weld the resins by laser, a laser beam transmissive resin is superposed on an upper surface of a laser beam absorbing resin, and a laser beam is irradiated from the side of the laser beam transmissive resin to melt the resins at an interface therebetween, thereby performing welding. In this case, in order to allow the laser to pass through the laser beam transmissive resin and appropriately heat a welded portion at the interface between the laser beam transmissive resin and the laser beam absorbing resin, it is necessary to apply an appropriate amount of heat to the welded portion without excess or deficiency. For this purpose, it is important to know transmittance of the laser beam transmissive resin among the resins to be welded.

Therefore, in an invention described in Patent Literature 1 shown in Fig. 12, before laser welding is performed, a measurement light source 109 irradiates a transmissive material 104 with a light beam 111 through an optical fiber 112 and an optical system 110 in advance. An amount of the light beam 111 passes through the transmissive material 104 is measured by a detector 108, and thus transmittance of the transmissive material 104 is calculated by a calculation device 106. Output of a laser beam source 101 is controlled based on the transmittance.

As a result, intensity of a laser beam 103 that is emitted from the laser beam source 101 to a welded portion via the optical fiber 107 and the optical system 102 is changed in magnitude corresponding to the transmittance of the transmissive material 104. Therefore, since energy whose magnitude is not excessive or insufficient is applied to a surface of an absorptive material 105 of the welded portion, heat generation having appropriate magnitude is achieved on the surface of the absorptive material 105, and thus laser welding can be performed with high quality.

In addition, in an invention described in Patent Literature 2 shown in Fig. 13, division by laser is performed instead of welding by laser. Here, in order to perform laser machining, separately from a first laser beam 245a irradiated from an incident surface Pu of a board P toward an emission surface Pd via a lens 246a, a transmitted light amount of a second laser beam 245b irradiated from an oblique direction to a material modified portion 247 of the board P via a lens 246b is detected by a light receiving element 2151, which is a transmitted light amount detection unit, and thus transmittance of the second laser beam 245b is calculated. As a result, it is possible to determine whether the material modified portion 247 is formed inside the board P with high accuracy. As a result, the board P is divided after determining whether the material modified portion 247 is formed inside the board P, and thus division failures can be prevented.

According to the inventions described in Patent Literature 1 and Patent Literature 2, appropriate laser machining can be performed by measuring laser beam resin transmittance in advance.

### CITATION LIST

### PATENT LITERATURE

Patent Literature 1: JP-A-2004-243629
Patent Literature 2: JP-A-2007-319881

### SUMMARY OF INVENTION

### TECHNICAL PROBLEM

Such laser resin welding is a technique used in a large number of industries and fields, and a variety of resin materials are used for welding in an overlapping manner. As one of such materials, a fire retardant resin material obtained by adding a fire retardant to a resin is often used. In the fire retardant resin, since the fire retardant is added as an impurity, a laser beam is diffused by the fire retardant, and thus laser beam resin transmittance is lowered.

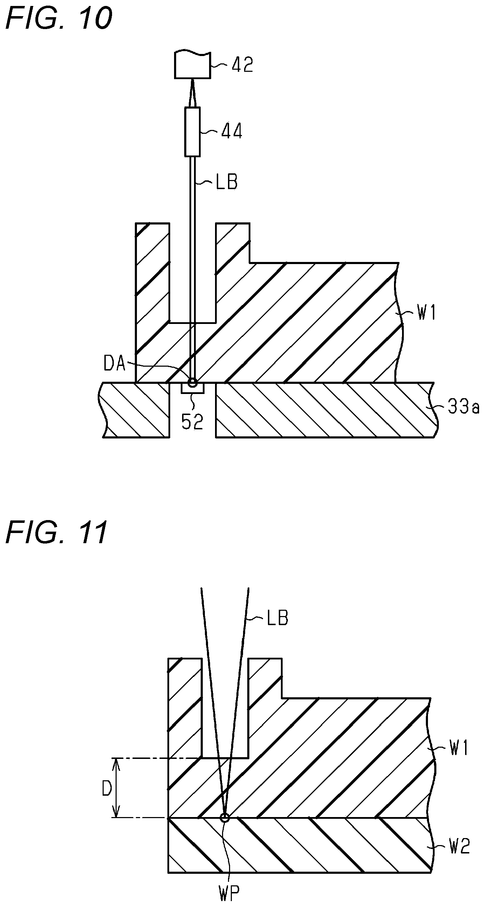

Therefore, as shown in Fig. 11, a narrow groove-shaped portion is formed in a laser beam transmissive resin W1 in a welded portion WP of an interface between the laser beam transmissive resin W1 and a laser beam absorbing resin W2 at the time of laser resin welding. In this way, a thickness D of the laser beam transmissive resin W1 may be reduced to facilitate transmission of a laser beam LB.

In Patent Literature 1, the light beam 111 is emitted from the measurement light source 109 to the transmissive material 104, and the amount of the light beam 111 passing through the transmissive material 104 is measured by the detector 108 that is provided separately below. In this case, when transmittance is low, the amount of light may be insufficient, and when the measurement light beam is a light beam whose diameter is thick, like a halogen light source, the narrow groove-shaped portion may not be accurately measured.

In Patent Literature 2, a material modification state can be recognized based on the amount of the transmitted laser beam 245b. However, the laser beam 245b is irradiated from an angle different from the machining laser beam 245a at a place different from an actual machining surface. Further, since the light receiving element 2151 is separated from the machining surface, the transmittance may not be accurately measured. In addition, when irradiation is performed obliquely, the narrow groove-shaped portion cannot be accurately measured.

Therefore, in order to accurately measure the transmittance, a large spectrometer is used. By such a spectrometer, even a fire-retardant resin that has a small light beam width, a wavelength close to that of the machining laser beam, and low light resin transmittance due to large output can be accurately measured.

However, in such a large-sized spectrometer, it is difficult to easily and quickly measure laser beam resin transmittance by incorporating the spectrometer in a production line, in particular, because the equipment is large and it is necessary to prepare a test piece conforming to a specification thereof.

The present disclosure has been made in view of the above circumstances, and an object thereof is to provide a photoelectric sensor capable of accurately measuring laser beam resin transmittance with a simple configuration, a method for measuring resin transmittance in laser resin welding, a laser resin welding method, and a laser machining device.

### SOLUTION TO PROBLEM

A photoelectric sensor according to the present disclosure includes: a placement table configured to allow a workpiece to be placed thereon; light projecting means including a light emitting element configured to emit light and converging means for converging the light emitted from the light emitting element toward a detection area; and light receiving means for receiving the light passing through the detection area from the converging means at a position located on a same plane as the placement table in a direction along an optical axis of the light, in which the optical axis of the light projected from the light projecting means is set such that the light is incident in a direction perpendicular to an incident surface of the light receiving means and focused on the incident surface of the light receiving means.

A photoelectric sensor according to another aspect of the present disclosure includes: a placement table configured to allow a workpiece to be placed thereon; light projecting means including a light emitting element configured to emit light and collimating means for converting the light emitted from the light emitting element after convergence into parallel light toward a detection area; and light receiving means for receiving the light passing through the detection area from the collimating means at a position located on a same plane as the placement table in a direction perpendicular to an optical axis of the light, in which the optical axis of the light emitted from the light projecting means is set such that the light is incident in a direction perpendicular to an incident surface of the light receiving means.

In the photoelectric sensor, the light emitting element may be a laser diode, the photoelectric sensor may further include an optical fiber having a core diameter smaller than a light emitting area of the laser diode, and light emitted by the light projecting means via the optical fiber and received by the light receiving means is smaller than the light emitting area of the laser diode.

In this case, the optical fiber may be configured as a single-mode fiber.

The photoelectric sensor may include a display unit configured to display resin transmittance of a measured resin.

A method for measuring resin transmittance in laser resin welding according to the present disclosure includes: a step of preparing any one of the photoelectric sensors described above; a step of placing only a laser beam transmissive resin configured to allow a laser to pass therethrough, which is included in a plurality of resins to be laser-welded, on the incident surface of the light receiving means on a surface where the laser beam transmissive resin is to be welded; and a step of measuring resin transmittance, which is laser beam transmittance of the laser beam transmissive resin.

A laser resin welding method according to the present disclosure includes: a step of preparing the photoelectric sensor including the display unit; a step of placing a laser beam transmissive resin configured to allow a laser to pass therethrough, which is included in a plurality of resins to be laser-welded, on the placement table of the photoelectric sensor; a step of measuring resin transmittance which is laser beam transmittance of the laser beam transmissive resin; a step of setting a machining condition of a laser machining device based on the resin transmittance displayed on the display unit; and a step of performing laser welding of the laser beam transmissive resin by the laser machining device under the set machining condition.

A laser machining device according to the present disclosure includes: any one of the photoelectric sensors described above; and a laser machining device body configured to emit a laser having a wavelength that is the same as or approximated to a wavelength of a laser emitted by the photoelectric sensor.

Further, a laser machining device according to another aspect of the present disclosure includes: any one of the photoelectric sensors described above; and a laser machining device body configured to receive data of laser beam resin transmittance measured by the photoelectric sensor and transmitted from the photoelectric sensor, and perform laser machining based on the data.

### ADVANTAGEOUS EFFECTS OF INVENTION

According to the photoelectric sensor, the method for measuring resin transmittance in laser resin welding, the laser resin welding method, and the laser machining device of the present disclosure, the laser beam resin transmittance can be accurately measured with a simple configuration.

### BRIEF DESCRIPTION OF DRAWINGS

Fig. 1 is a schematic view showing an entire laser machining device according to a first embodiment.
Fig. 2 is a schematic view showing a principle of resin welding by laser beam in the first embodiment.
Fig. 3 is a perspective view showing an appearance of a photoelectric sensor according to the first embodiment.
Fig. 4 is a perspective view of the photoelectric sensor according to the first embodiment from which a cover is removed.
Fig. 5 is an exploded perspective view of light projecting means of the photoelectric sensor according to the first embodiment.
Fig. 6 is an exploded perspective view of light receiving means of the photoelectric sensor according to the first embodiment.
Fig. 7 is a block diagram showing a configuration of the photoelectric sensor according to the first embodiment.
Fig. 8 is a schematic view showing a method for measuring resin transmittance according to the first embodiment.
Fig. 9 is a schematic view showing a modification of the method for measuring resin transmittance according to the first embodiment.
Fig. 10 is a schematic view showing a method for measuring resin transmittance according to a second embodiment.
Fig. 11 is a schematic view showing an example of resin welding by laser beam according to the present embodiment.

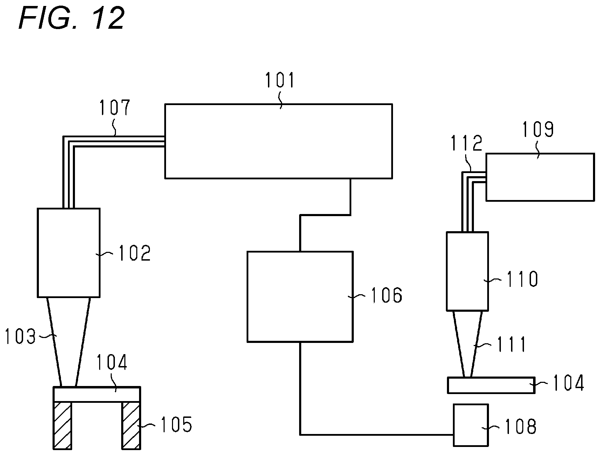

Fig. 12 is a schematic view showing a photoelectric sensor according to related art.

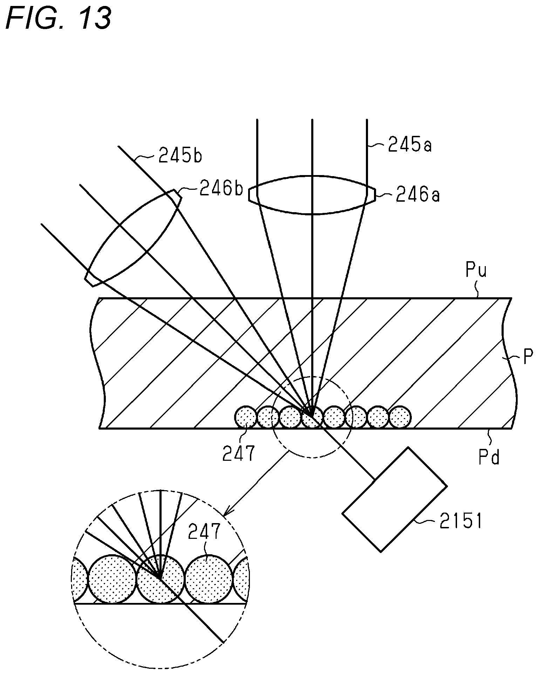

Fig. 13 is a schematic view showing a photoelectric sensor according to related art.

### DESCRIPTION OF EMBODIMENTS

### (First Embodiment)

### <Overall Configuration of Laser Machining Device 1>

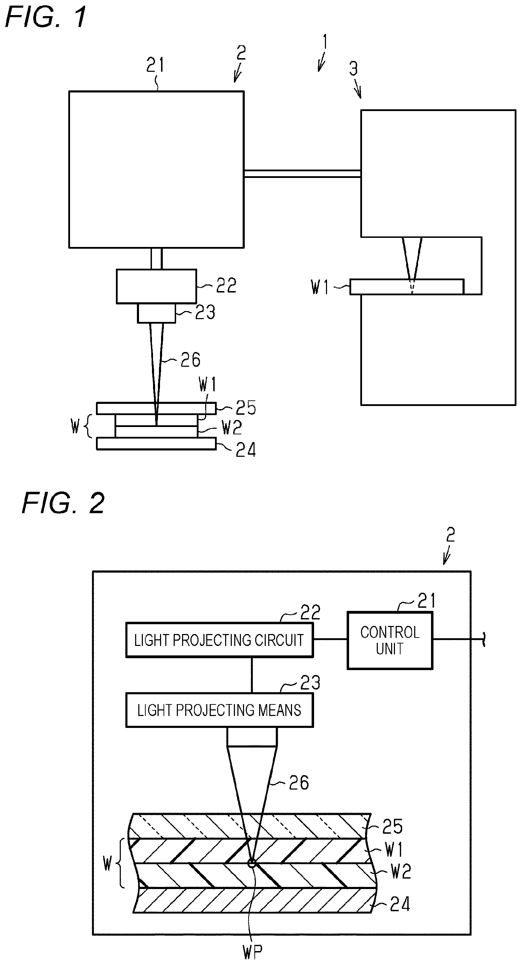

Fig. 1 is a schematic view showing a configuration of a laser machining device 1 according to the present embodiment. Fig. 2 is a schematic view showing a principle of resin welding by laser light. As shown in Fig. 1, the laser machining device 1 includes a laser machining device body 2 and a photoelectric sensor 3.

In laser resin welding (laser welding), as shown in Fig. 2, a workpiece W including a workpiece W1 made of a laser beam transmissive resin and a workpiece W2 made of a laser beam absorbing resin is used. In the photoelectric sensor 3, laser beam transmittance T (hereinafter, referred to as "resin transmittance T") of the laser beam transmissive resin of the workpiece W1 is measured in advance using only the workpiece W1, and data thereof is sent (transmitted) to the laser machining device body 2 as the resin transmittance T in laser resin welding, and the laser machining device body 2 performs the laser resin welding upon receiving the data of the resin transmittance T.

### <Overall Configuration of Laser Machining Device Body 2>

The laser machining device body 2 is, for example, a known laser machining device, and three-dimensionally irradiates the workpiece W placed on a stage 24, which is an example of a workpiece placement table, with a laser beam 26 to perform cutting, drilling, and welding. In the laser machining device body 2, the workpiece W2 is placed on the stage 24, the workpiece W1 is overlapped thereon and fixed by a glass plate 25. The control unit 21 transmits a control signal to a light projecting circuit 22, which is a driver, based on the received data of the resin transmittance T, and the light projecting circuit 22 optimizes output of the laser beam 26 and transmits a drive signal to light projecting means 23. When the laser beam 26 is emitted by the light projecting means 23, the laser beam 26 substantially passes through the glass plate 25, and then passes through the workpiece W1, which is the laser beam transmissive resin, while being scattered and attenuated at a certain rate. The laser beam 26 is hardly converted into heat by the glass plate 25, and the conversion from light to heat is also restrained in the workpiece W1 that is made of the laser beam transmissive resin. Therefore, the workpiece W1 is not melted by heat. When the laser beam 26 reaches the workpiece W2 made of the laser beam absorbing resin, the workpiece W2 absorbs the laser beam 26 and converts the laser beam 26 into heat. Then, the workpiece W2 is melted by the heat, the heat is conducted to the workpiece W1, the temperature of the workpiece W1 is increased, and an interface portion between the workpiece W1 and the workpiece W2 is melted. Therefore, the melted workpiece W2 and workpiece W1 are mixed with each other, and are welded when cooled and solidified.

### <Fire Retardant Resin>

As the laser beam resin transmittance T increases, less heat is generated in the workpiece W1, and the laser resin welding is performed more easily, so that the laser beam resin transmittance T is required to some extent. Further, in order to perform welding, the resin is required to be a heat-meltable resin that is melted by heating and solidified by cooling.

Here, examples of the transmissive resin material, which is one of a plurality of resin materials that can be bonded by this welding method, include the following. Resin materials having good transmittance, such as polypropylene (PP), polycarbonate (PC), acrylonitrile butadiene styrene copolymer (ABS), and polyamide (PA). In addition, as a material having relatively low transmittance, a material such as polybutylene terephthalate (PBT) or polyphenylene sulfide (PPS) is used.

In the present embodiment, a fire-retardant resin is exemplified as the laser beam transmissive resin used for the workpiece W1. Examples of the fire retardant resin include a fluorine resin such as tetrafluoro ethylene, and silicone rubber. However, since the laser beam resin transmittance T is low, fire retardancy may be secured by kneading a fire retardant filler such as aluminum hydroxide or magnesium hydroxide into a resin having high transmittance. In this case, since the fire retardant filler does not allow the laser beam to pass therethrough, the laser beam 26 incident on the workpiece W1 hits the filler inside and is scattered, and is greatly attenuated before reaching a welded portion WP. Therefore, it is necessary to adjust the output of the laser beam 26 based on the resin transmittance T of the laser beam 26 through the workpiece W 1.

### <Control Unit 21, Light Projecting Circuit 22, and Light Projecting Means 23 of Laser Machining Device Body 2>

Therefore, in the laser machining device 1 of the present embodiment, the control unit 21 calculates an attenuation rate of the laser beam 26 based on the resin transmittance T, and issues a light projection output instruction to the light projecting circuit 22. The light projecting circuit 22, which is the driver, applies predetermined electric power to the light projecting means 23 to emit the laser beam 26.

In the present embodiment, in order to perform sufficient welding, it is desirable that the laser beam resin transmittance T is approximately 30% or more. Therefore, by determining, for example, whether the resin transmittance T is 30% or more by the photoelectric sensor 3 on an upstream side of a production line, a welding failure can be checked before machining. Of course, the appropriate resin transmittance T varies depending on resin types, laser types, wavelengths, and the like.

### <Light Projecting Means 23 of Laser Machining Device Body 2>

In this embodiment, Yb:fiber laser having a wavelength of 1070 nm is used as the light projecting means 23. Examples thereof include a laser oscillator such as VL-W1A00 manufactured by Panasonic Corporation. The laser oscillator emits the laser beam 26 that has a fundamental wavelength (1070 nm) through a predetermined optical system. In this case, a position of a focal point S of the laser beam 26 in a Z direction is determined to be aligned with the welded portion WP at the interface between the workpiece W1 and the workpiece W2. For example, when the light projecting means 23 includes an optical system that adjusts a focal length, the position of the focal point S of the laser beam 26 in the Z direction is adjusted by adjusting the focal length to align with the welded portion WP. In addition, the position of the focal point S of the laser beam 26 in the Z direction may be aligned with the welded portion WP by adjusting a relative position between the light projecting means 23 and the stage 24.

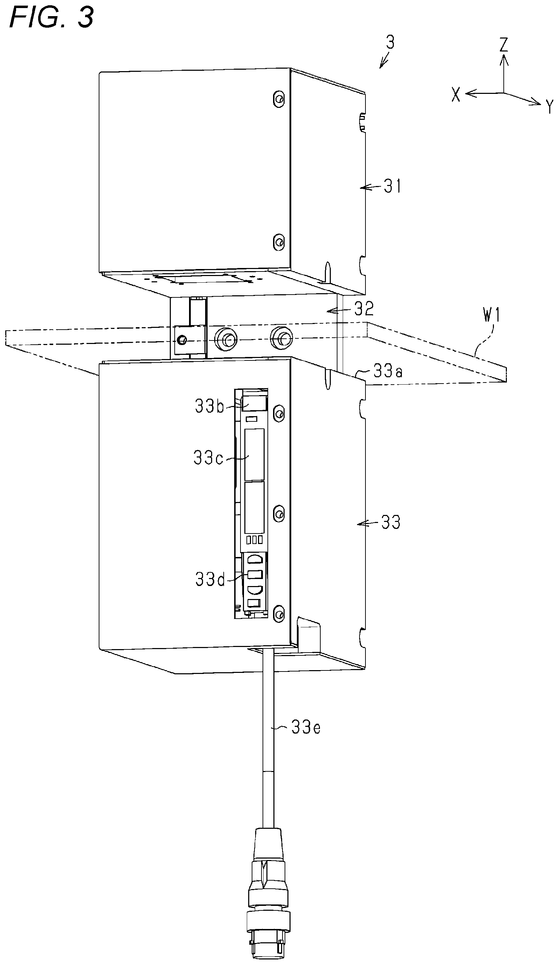

As shown in Fig. 1, the photoelectric sensor 3 is connected to the laser machining device body 2. Next, the photoelectric sensor 3 will be described with reference to Figs. 3 to 8.

### <Overall Configuration of Photoelectric Sensor 3>

Fig. 3 is a perspective view showing an appearance of the photoelectric sensor 3. The photoelectric sensor 3 is provided with a plate-shaped column 32 on a back surface side (right back side of Fig. 3), which is a frame of the entire photoelectric sensor 3, and is provided with a rectangular parallelepiped box-shaped upper housing 31 on an upper portion thereof. Below the upper housing 31, a lower housing 33 that has substantially the same width and depth as those of the upper housing 31 and is larger in a height direction is disposed at a distance from the upper housing 31. An upper surface of the lower housing 33 serves as a placement table 33a. Here, only the workpiece W1 made of the laser beam transmissive resin is placed with the welded portion WP facing downward.

On a front surface of the lower housing 33, there are provided a display lamp 33b constituted by an LED or the like for displaying an action, a display unit 33c provided with a liquid crystal screen for displaying characters, and an operation switch 33d provided with buttons for operation. A connection cable 33e configured to connect the laser machining device body 2 extends from a lower surface of the lower housing 33.

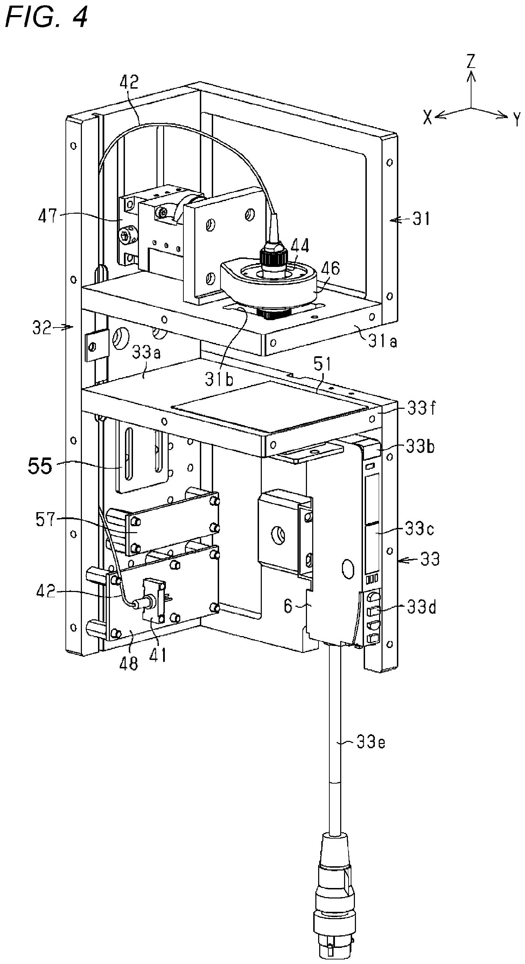

Next, inside of the photoelectric sensor 3 will be described with reference to Fig. 4. Fig. 4 is a perspective view of the photoelectric sensor 3 in a state where a cover is removed for the purpose of describing the inside. The column 32 is disposed to extend from an upper end to a lower end on the back surface side (left back side in Fig. 4).

### <Internal Structure of Upper Housing 31>

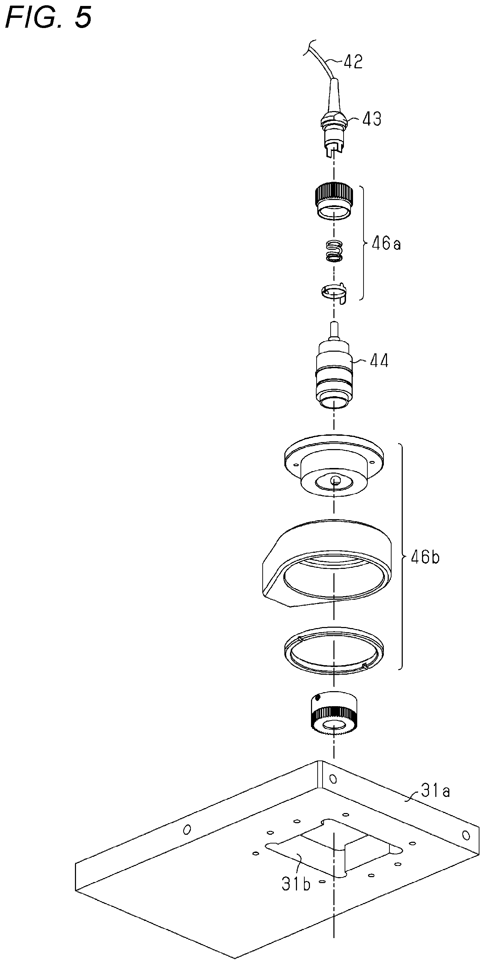

Apart of the light projecting means 4 is disposed inside the upper housing 31. A horizontal plate-shaped base 31a is provided on the column 32 at the lower end, and a substantially rectangular opening 31b is formed in the base 31a so as to penetrate in a vertical direction on a slightly front side. A collimator lens 44 constituting the light projecting means 4 is fixed to the opening 31b by a fixing tool 46 constituted by a plurality of elements. The fixing tool 46 includes fixing tools 46a and 46b (see Fig. 5), and is supported by an XYZ moving stage 47 fixed to the column 32. The XYZ moving stage 47 can be slightly moved in an X direction (horizontal left-right direction parallel to the column 32) and a Y direction (horizontal front-rear direction perpendicular to the column 32), and adjusts a position of an optical axis of the laser beam in an XY plane (incident surface) relative to the light receiving means 5. In addition, the XYZ moving stage 47 can be slightly moved in a Z direction (direction along the optical axis), and can adjust the beam to converge to one point relative to the light receiving means 5. In particular, when a refractive index of the workpiece W1 is different, adjustment can be performed in such a manner that the beam is correctly converged on the welded portion WP.

It should be noted that movement in the XY direction and the Z direction may be omitted.

### <Internal Structure of Lower Housing 33>

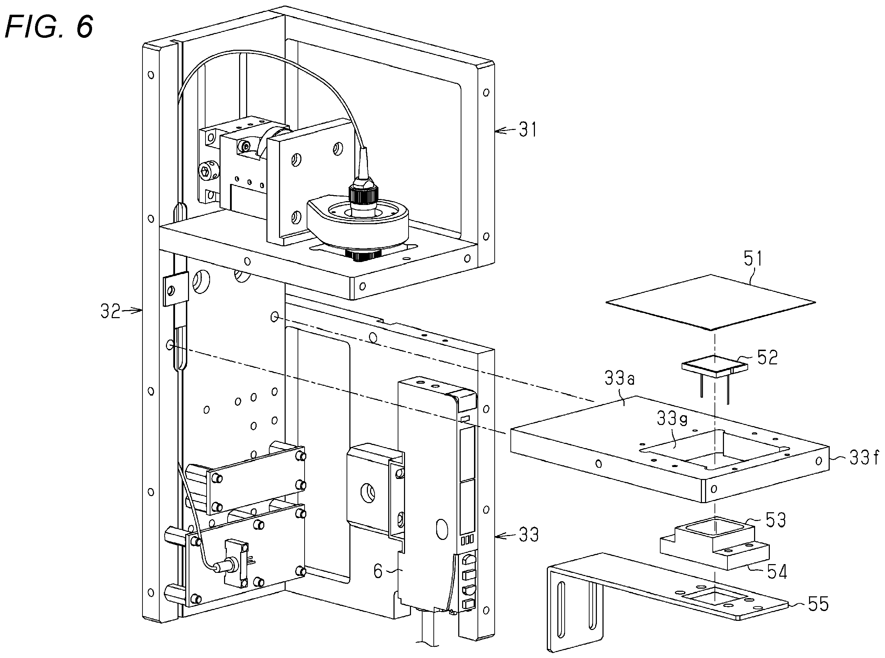

In the lower housing 33, a plate-shaped window frame 33f that constitutes the placement table 33a disposed at an upper end of the lower housing 33 is horizontally supported by the column 32. In the window frame 33f, a substantially rectangular window 33g (see Fig. 6) is opened at a position facing the opening 3 1b of the upper housing 31 in the vertical direction. The light receiving means 5 is disposed in the window 33g.

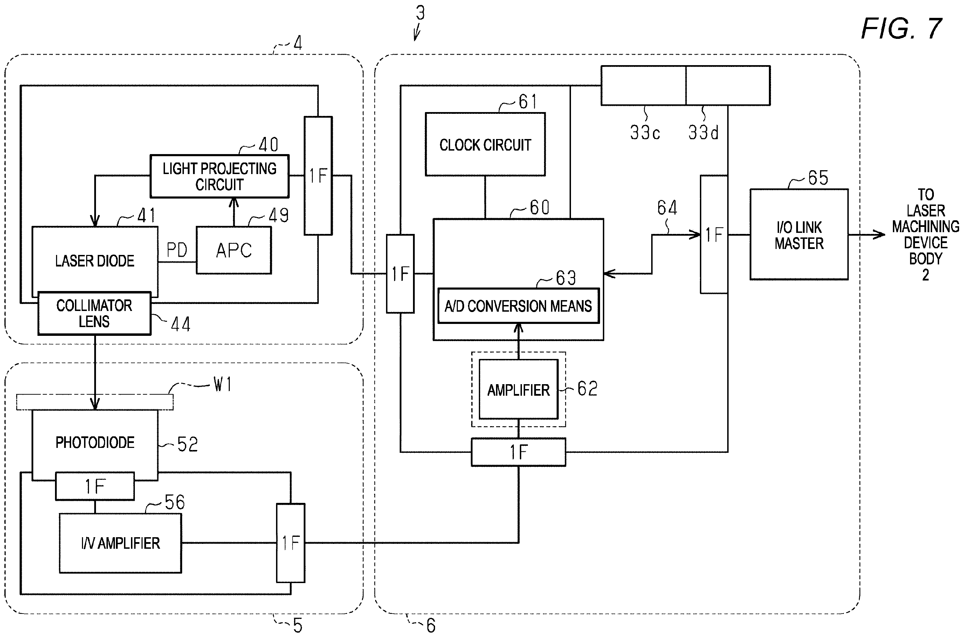

A light receiving board 57 constituting the light receiving means 5 and a light projecting board 48 constituting the light projecting means 4 are disposed on a lower inner side of the column 32. A laser diode 41 (hereinafter, also referred to as the "LD 41") is disposed on the light projecting board 48, and the laser beam oscillated here is led out to the upper housing 31 along the column 32 by an optical fiber cable 42. Control means 6 including a control board 60 (see Fig. 7) is disposed inside a back side of the display unit 33c. The optical fiber cable 42 is a single-mode optical fiber cable, and a core diameter of the optical fiber cable 42 of the present embodiment is 5.3 µm.

### <Light Projecting Means 4>

Fig. 5 is an exploded perspective view of a part of the light projecting means 4. Fig. 7 is a block diagram showing an electric configuration of the photoelectric sensor 3.

The light projecting means 4 receives a control signal from the control board 60 of the control means 6, causes the LD 41, which is an example of a light emitting element of the light projecting means 4, to emit light by the light projecting circuit 40, which is a driver, and projects the light through an optical system such as the collimator lens 44.

Here, a configuration in which the LD 41 is caused to emit light and the light is projected via an optical system such as the collimator lens 44 will be described with reference to Fig. 5.

First, as shown in Fig. 4, the light projecting board 48, which is a driver, receives a control signal from the control means 6, and then the light projecting board 48 supplies a drive current to the LD 41 to cause the LD 41 to oscillate. A laser beam oscillated by the LD 41 is guided to the collimator lens 44 by the optical fiber cable 42, and is irradiated to a detection area which is an incident surface of a photodiode 52.

### <LD (Laser Diode) 41>

The LD 41, which is an example of the light emitting element, has a predetermined light emitting area. In the light emitting area of the LD 41 of the present embodiment, the core diameter of the optical fiber cable 42 is larger than 5.3 µm, and a shape of the light emitting area is basically rectangular. A diameter of the light emitting area of the LD 41 of the present embodiment is 1.01 mm to several mm. By allowing light to pass through the optical fiber cable 42, a light beam having a uniform circular cross-sectional is obtained. In addition, emission from the optical fiber cable 42 is close to a point light source. That is, the laser beam LB emitted from the LD 41 is shaped by the predetermined optical fiber cable 42.

As the LD 41, for example, a Fabry-Perot semiconductor laser (Fabry-Perot laser diode) or the like is preferably used, a oscillation mode thereof is a single mode, and a wavelength thereof is λ = 1064 nm.

Since the laser beam emitted from the laser oscillator (light projecting means 23) of the laser machining device body 2 has a wavelength of λ = 1070 nm, the wavelength λ is not exactly the same as the wavelength of the laser beam emitted by the LD 41, but is a approximated wavelength. The resin transmittance T of the laser beam transmissive resin constituting the workpiece W1 is greatly changed relative to the wavelength. Therefore, even when the resin transmittance T is measured by the photoelectric sensor 3 with infrared light having a wavelength greatly different from the wavelength of the laser beam emitted from the laser machining device body 2, for example, a wavelength λ of 780 to 850 nm, the resin transmittance T at the time of welding in the laser machining device body 2 cannot be measured since different resin transmittance T is exhibited in the laser machining device body 2.

In the present embodiment, when the resin transmittance T is measured at λ = 1064 nm, even if the machining is performed at λ = 1070 nm, the machining can be performed with an allowable error of the resin transmittance T. In addition, by allowing such an error which is not problematic in practice, it is possible to manufacture the photoelectric sensor 3 at a lower cost by using an inexpensive laser diode. That is, even though the wavelength of the laser beam of the light projecting means 23 and the wavelength of the laser beam of the LD 41 are not exactly the same, the wavelength of the laser beam of the light projecting means 23 may be different from the wavelength of the laser beam of the LD 41 while having a wavelength close to the wavelength of the laser beam of the LD 41 as long as an error of the resin transmittance T measured at each wavelength is within a practically allowable range. The "wavelength close to (not exactly the same as) the wavelength of the laser beam of the LD 41" may be interpreted as being included in the same range as the wavelength of the laser beam of the LD 41.

### <Optical Fiber Cable 42>

An end surface of the optical fiber cable 42 is located at a position facing the light emitting area of the LD 41, and the laser beam is introduced therefrom. As described above, the diameter of the light emitting area of the LD 41 is 1.01 mm to several mm, whereas the core diameter of the optical fiber cable 42 is 5.3 µm. Therefore, the laser beam oscillated by the LD 41 is converged by a condenser lens and is input from an end portion of the optical fiber cable 42. The optical fiber cable 42 may be a step-index multi-mode optical fiber (SI), a graded-index multi-mode optical fiber (GI), or the like depending on the purpose, and, in the present embodiment, a general-purpose single-mode optical fiber (SM) is adopted. This single-mode optical fiber is an optical fiber having one mode by reducing the core diameter, and distortion of a propagated signal does not occur due to differences between modes as seen in multi-mode fibers, and thus single-mode optical fiber has extremely wide band characteristics. Since the general-purpose single-mode optical fiber has a zero-dispersion wavelength in a 1310 nm band, transmission loss is low and thus the optical fiber has excellent characteristics. Therefore, the focal point S of the laser beam LB oscillated by the LD 41 can be accurately concentrated on a detection area DA, which is the incident surface of the photodiode 52, without deviation of the focal point S.

Specifically, the optical fiber cable 42 is a single-mode optical fiber cable, and the core diameter of the optical fiber is 5.3 µm in the embodiment.

### <Optical Connector 43>

The optical fiber cable 42 is guided from the light projecting board 48 of the lower housing 33 to the upper housing 31 along the column 32. A screw-type FC optical connector 43 is connected to the end portion of the optical fiber cable 42. As shown in Fig. 5, ferrules are pressed against each other by a spring, brought into close contact with each other by the fixing tool 46a and connected to the collimator lens 44 which is an example of collimating means. At the end portion of the optical fiber cable 42, the laser beam propagated by using a core emits a spherical wave in a divergent manner. Ultra PC (UPC) polishing, during which reflection at a connection portion is reduced by polishing an end surface of a ferrule into a spherical surface such that fiber cores are closely adhered to each other, is performed.

### <Collimator Lens 44 (Collimating Means)>

The collimator lens 44 is supported by the fixing tool 46b at a predetermined position on the column 32, and converges the spherical wave of the laser beam into parallel light. The collimator lens 44 can be suitably implemented by, for example, an adjustable collimator lens with an aspherical lens (f = 2.0 mm).

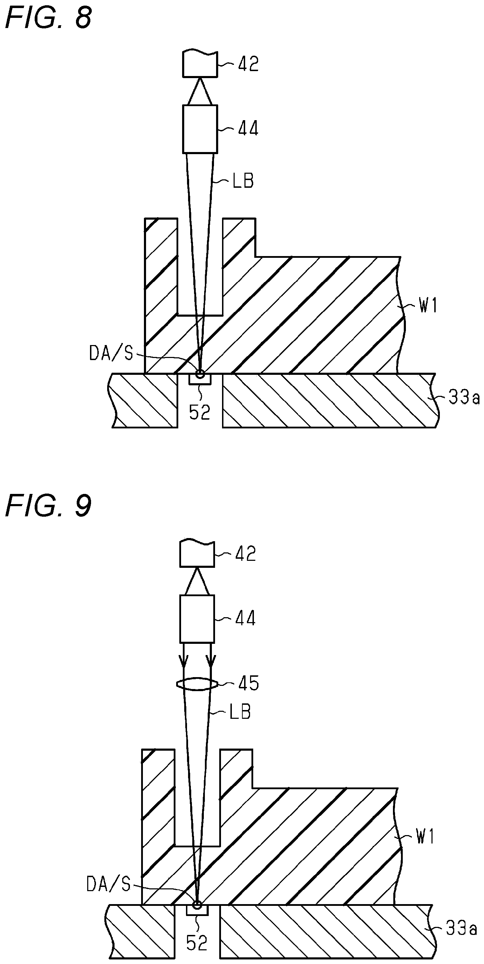

In the collimator lens 44 of the present embodiment, the light is converged by about 0.3° from the parallel light. Further, according to the collimator lens 44 of the present embodiment, since the collimator lens 44 includes a focus mechanism, this focus mechanism may be used. In this case, it can be said that the collimator lens 44 also has a function of a converging lens 45 (see Fig. 9).

As shown in Fig. 8, in the present embodiment, the laser beam LB passing through the collimator lens 44 forms the focal point S in the detection area DA of the incident surface of the photodiode 52. That is, the detection area DA, the focal point S, and further the welded portion WP are points that coincide with each other.

In addition, in a state where the workpiece W1 made of the laser beam transmissive resin is placed, deviation of the focal point S caused by refraction when passing the resin is adjusted, and thus focusing is performed more accurately.

### <Light Receiving Means 5>

Fig. 6 is a partially exploded perspective view of the photoelectric sensor 3. The light receiving means 5 will be described with reference to Fig. 6. The window 33g of the window frame 33f (see Fig. 4) is covered with a transparent conductive film 51 that is transparent, thin and made of resin. The photodiode 52 is disposed below the transparent conductive film 51 via an adhesive at a position where the incident surface is in close contact with the transparent conductive film 51. That is, the incident surface of the photodiode 52 is located on the same plane as the placement table 33a. The photodiode 52 is connected and fixed to a PD board 54 accommodated in a square fixing portion 53, and is fixed to the column 32 by an L-shaped metal fitting 55. The PD board 54 is connected to the light receiving board 57 by a conductive wire (not shown).

### <Photodiode 52>

The photodiode 52 is adapted to the wavelength of the LD 41, which is an example of the light emitting element of the light projecting means 4. The photodiode 52 of the present embodiment is a Si photodiode that has sensitivity at a wavelength of 190 to 1100 nm, and corresponds to the wavelength of 1064 nm of the LD 41 of the light projecting means 4.

### (Operation of Present Embodiment)

### <Measurement of Resin Transmittance T>

Next, a method for measuring resin transmittance of the present embodiment will be described with reference to Fig. 8. When the spherical wave of the laser beam LB is emitted from the end of the optical fiber cable 42, the spherical wave is incident on the collimator lens 44. When the laser beam LB is incident on the collimator lens 44, the laser beam LB is converged by about 0.3° relative to the parallel light, and becomes converged light. Then, the laser beam LB is incident on the workpiece W1 made of the laser beam transmissive resin. The laser beam LB is diffused and attenuated due to the fire-retardant filler, and the focal point S is focused on the incident surface of the photodiode 52, that is, the detection area DA. The detection area DA coincides with the welded portion WP shown in Fig. 11. Therefore, according to the method for measuring the resin transmittance of the present embodiment, it is possible to accurately estimate thermal energy received by the actual welded portion WP.

When the laser beam LB is the converged light, a light beam through which the laser beam LB passes has a certain area. Therefore, even if the laser beam LB of a specific path is diffused by the fire-retardant filler and does not reach the detection area DA, the laser beam LB of another path passes through the workpiece W1 and reaches the detection area DA. Therefore, an average of the laser beams LB of a large number of paths is measured, and thus a variation in measurement values is reduced.

### <Control Means 6>

Next, an electric configuration and operation of the control means of the photoelectric sensor 3 will be described with reference to Fig. 7.

The photoelectric sensor 3 includes the control board 60 constituting the control means 6. The control board 60 has a configuration of a known computer, and controls the photoelectric sensor 3 with reference to a clock circuit 61. Specifically, a control signal is sent to the light projecting circuit 40 placed on the light projecting board 48 of the light projecting means 4. Upon receiving the control signal, the light projecting circuit 40 applies a predetermined voltage to the LD 41 to cause the LD 41 to emit light. The light projecting circuit 40 monitors the laser beam LB with a photodiode (not shown), performs feedback, and adjusts the laser beam LB to have a constant light amount by an automatic power control (APC) 49.

The laser beam emitted from the LD 41 passes through the workpiece W1 and incident on the photodiode 52. With respect to the laser beam incident on the photodiode 52, a current corresponding to the incident light is converted from a current I to a voltage V by an I/V amplifier (current/voltage conversion circuit) 56. Thereafter, the voltage V is converted into a digital signal by A/D conversion means 63 of the control board 60 via an amplifier 62, and the digital signal is sent to the control circuit of the control board 60. On the control board 60, the resin transmittance T of the workpiece W1 is calculated by comparing the received digital signal with a reference value corresponding to resin transmittance of 100% which is measured and set in advance by teaching in a state where the workpiece W1 is absent.

The reference value may need to be corrected depending on deterioration of the LD 41, contamination of the optical system, and temperature conditions. Therefore, the reference value may be set in advance and corrected by calibration at any time, or may be set by teaching without the workpiece W1 each time.

### <Display Means>

The resin transmittance T is displayed on the display unit 33c. The display unit 33c is configured to be capable of displaying characters by a liquid crystal panel, and the resin transmittance is displayed by characters.

### <IO-Link>

Data of the resin transmittance T is transmitted to an I/O link master 65 via an I/O link 64.

An IO-Link is an industrial interface standard (IEC61131-9) designed for the purpose of connecting a sensor or an actuator to a field bus and an industrial Ethernet (registered trademark) system by point-to-point bi-directional digital communication.

By communicating operation states and identification information of the sensor or the actuator with a control device (PLC), states and information on the individual sensor or actuator in a factory, which is manually managed conventionally, can be provided to an administrator. Accordingly, this is a technique for achieving system construction that enables automation of factory system management.

The I/O link master 65 includes a plurality of ports, one device can be connected to one port, and thus communication with a plurality of devices can be performed at the same time. Therefore, the data is transferred to the laser machining device body 2 via the I/O link master 65. Upon receiving the data, the laser machining device body 2 calculates, in the control unit 21, machining data (machining condition) for welding the workpiece W1. Examples of the machining data include output of the laser beam emitted from the light projecting means 23, an irradiation time of the laser beam, a repetition frequency (number of pulses) of the laser irradiation, a relative movement speed between the light projecting means 23 and the stage 24, and a combination thereof. In addition, although not shown, in a case where the light projecting means 23 includes scanning means, a scanning speed of the laser beam is also exemplified as the machining data (machining condition) as a relative movement speed.

### (Effects of Present Embodiment)

According to the laser machining device 1 of the above embodiment, the following effects can be obtained.

(1) In the above embodiment, in the laser resin welding of the laser machining device 1, since the resin transmittance T of the laser beam transmissive resin to be machined by the photoelectric sensor 3 can be measured before welding, the laser resin welding can be performed with appropriate output.

(2) In particular, the photoelectric sensor 3 is disposed on the upstream side of the production line. The resin transmittance T is measured in advance at the position where the workpiece W1 to be laser-welded is machined. Then, if machining is performed at that position on a downstream side of the production line, high-quality laser resin welding can be performed extremely efficiently in an in-line manner without waiting time.

(3) The photoelectric sensor 3 used for the measurement can have a simple and inexpensive configuration using the LD 41.

(4) Since the laser beam emits the measurement light beam by making the core diameter of the optical fiber smaller than the diameter of the light emitting area of the LD 41, the welded portion formed in the groove shape can be accurately measured.

(5) The laser beam is converged by using the converging lens 45, passes through the workpiece W1 which is the laser beam transmissive resin, and the focal point S is focused on the incident surface of the photodiode 52 which is the light receiving means. Therefore, since the resin transmittance is measured in the detection area DA at the same position as the actual welded portion WP, measurement can be performed accurately.

(6) In particular, by using the converged light, unlike a straight laser beam, the converged light is incident on the workpiece W1 in a wide range as a path, so that the resin transmittance T can be accurately measured even when the resin transmittance T is low in a resin, such as a fire-retardant resin containing a non-flammable filler that does not allow the laser beam to pass therethrough.

(7) Since the wavelength of the measurement LD 41 is equivalent to the wavelength of the laser for laser machining, transmission characteristics of the laser beam transmissive resin can be correctly measured according to the wavelength of the laser beam.

(8) Since the measurement laser beam is measured on the same path as the machining laser beam, an accurate measurement result can be obtained.

(9) In addition, by using the I/O link master 65, factory production management can be automated based on the resin transmittance T measured by the photoelectric sensor 3.

(10) Since the display unit is provided and the measured resin transmittance T can be displayed, the photoelectric sensor 3 can be used offline. That is, output of the laser machining device body 2 can be made appropriate by manually dividing the output of the laser machining device body 2 by the resin transmittance T in accordance with the resin transmittance T measured here.

(11) Therefore, since the photoelectric sensor 3 of the present embodiment can be easily externally attached or retrofitted, the photoelectric sensor 3 can be easily introduced even to an existing production facility.

### (Modifications) The above embodiment may be modified as follows.

### <Converging Lens 45>

Fig. 9 shows an example in which the projection optical system of the above embodiment is changed. In the above embodiment, the laser beam LB collimated by the collimator lens 44 is converged into the converged light by the collimator lens 44 itself that serves as the converging means. However, the collimator lens 44 is provided as originally collimating means for converting the laser beam LB into perfect parallel light, and is further provided with the converging lens 45. In addition, the focal point S of the laser beam LB converged by the converging lens 45 may be focused on the incident surface of the photodiode 52, that is, in the detection area DA.
- With such a configuration, the laser beam LB is caused to pass through the workpiece W1 in a wider range by causing the collimator lens 44 to once convert the light beam into parallel light having a large diameter and causing the converging lens 45 to converge the parallel light so as to focus the focal point S in a detection area DS. Therefore, the resin transmittance T of the workpiece W1 is more averaged, and thus measurement is performed accurately.
- In addition, it is possible to design to reduce diffusion, and a photodiode 52 having a smaller area can be used to achieve miniaturization.
- Further, when a thickness of an object is changed, it is necessary to re-adjust a convergence diameter, and it is also possible to increase power density and increase an S/N ratio.

### (Second Embodiment)

Next, the laser machining device 1 according to a second embodiment of the present disclosure will be described with reference to Fig. 10. Since the second embodiment has a configuration in which only the configuration of the light projecting means 4 of the first embodiment is changed, detailed description of the same parts will be omitted.

In the first embodiment, the converging lens 45 is provided downstream of the collimator lens 44 to convert the parallel light into the converged light. Then, the focal point S is focused in the detection area DA of the incident surface of the photodiode 52. In the present embodiment, the optical axis of the light emitted from the light projecting means 4 as linear parallel light obtained by reducing the light beam diameter of the laser beam LB is set by the collimator lens 44 in such a manner that the light is incident in a direction perpendicular to the incident surface of the photodiode 52.

Although not shown, it may be configured such that, once the collimator lens 44 is used to make the parallel light, the parallel light is converged again by the converging lens to obtain converged light, and a light beam diameter thereof is narrowed down, collimated with a concave lens or the like to obtain small diameter parallel light.

Therefore, according to the second embodiment, the following effects can be obtained in addition to the effects (1) to (4) and (7) to (11) described in the first embodiment.

(12) Since the laser beam LB is emitted from the light projecting means 4 as the linear parallel light having a small light beam diameter, a depth of focus is infinite, and thus adjustment of the focal point S is basically not necessary. Therefore, even if a distance between the light projecting means 4 and the light receiving means 5 is changed, the laser beam LB can be basically concentrated and measured at one point in the detection area DA, that is, on the welded portion WP. Therefore, when a size of the workpiece W1 is large, the measurement can be performed by increasing the distance between the light projecting means 4 and the light receiving means 5. In addition, when the workpiece W1 is thin, the distance between the light projecting means 4 and the light receiving means 5 is reduced such that positional accuracy of the laser beam LB can be improved while power attenuation can be reduced.

(13) In addition, even when a width of the groove-shaped portion provided in the welded portion WP of the workpiece W1 is narrow, there is no case where the resin transmittance T cannot be accurately measured due to interference with a wall surface of the groove-shaped portion.

(14) In the present embodiment, since it is possible to adopt a configuration in which the converging lens 45 is not used, the optical system can be further simplified, so that the configuration of the photoelectric sensor 3 can be further simplified.

### (Modification of Embodiment)

The above embodiment may be modified as follows.
∘ When a plurality of positions of the workpiece W1 are specified by image recognition and the resin transmittance T of the specified positions is measured, even when thicknesses of welded portions WP are different from each other, the welded portions WP can each be machined with appropriate laser output.
∘ The placement table 33a is not limited to be a flat surface extending around the photodiode 52, and may have the size of the photodiode 52. In other words, the photodiode 52 may be disposed on the same plane as a light receiving surface of the photoelectric sensor 3, and the placement table 33a may also serve as the light receiving surface. With such a configuration, even when there is a protruding portion below the workpiece W1, the welded portion WP can be prevented from being lifted from the photodiode 52.
∘ Although the LD 41 of the photoelectric sensor 3 of the embodiment is an Nd:YVO4 laser, a laser diode such as Nd:YAG (neodymium:yttrium-aluminum-garnet) having a wavelength approximated to the wavelength of λ = 1064 nm, for example, may be used as the light emitting element.

Although various embodiments have been described above with reference to the drawings, it is needless to say that the present disclosure is not limited to such examples. It will be apparent that those skilled in the art can conceive of various modifications and alterations within the scope described in the claims, and it is understood that such modifications and alterations naturally belong to the technical scope of the present disclosure. In addition, the respective constituent elements in the above-described embodiments may be combined as desired without departing from the gist of the disclosure.

The present disclosure is based on Japanese Patent Application No. 2019-118973 filed on June 26, 2019, the contents of which are incorporated herein as reference.

### REFERENCE SIGNS LIST

LB: measurement laser beam, T: resin transmittance (in laser resin welding), W: workpiece, W1: workpiece (laser beam transmissive resin), W2: workpiece (laser beam absorbing resin), D: thickness (of workpiece W1 at welded portion), WP: welded portion, DA: detection area, F: focal point, 1: laser machining device, 2: laser machining device body, 21: control unit, 22: light projecting circuit, 23: light projecting means, 24: stage, 25: glass plate, 26: laser beam, 3: photoelectric sensor, 31: upper housing, 31a: base, 32: column, 33: lower housing, 33a: placement table, 33b: display lamp, 33c: display unit capable of displaying characters, 33d: operation switch, 33e: connection cable, 4: light projecting means, 40: light projecting circuit, 41: LD (laser diode, light emitting element), 42: optical fiber cable, 43: optical connector, 44: collimator lens (collimating means), 45: converging lens (converging means), 46a, 46b: fixing tool, 47: XYZ moving stage, 48: light projecting board 48, 49: APC, 5: light receiving means, 57: light receiving board, 51: transparent conductive film, 52: photodiode (light receiving element), 53: square fixing tool, 54: PD board, 55: L-shaped metal fitting 55, 56: I/V amplifier, 6: control means, 60: control board, 61: clock circuit, 62: amplifier, 63: A/D conversion means, 64: I/O link, 65: I/O link master.

## Claims

1. A photoelectric sensor comprising:
a placement table configured to allow a workpiece to be placed thereon;
light projecting means comprising a light emitting element configured to emit light and converging means for converging the light emitted from the light emitting element toward a detection area; and
light receiving means for receiving the light passing through the detection area from the converging means at a position located on a same plane as the placement table in a direction along an optical axis of the light,
wherein the optical axis of the light projected from the light projecting means is set such that the light is incident in a direction perpendicular to an incident surface of the light receiving means and focused on the incident surface of the light receiving means.

2. A photoelectric sensor comprising:
a placement table configured to allow a workpiece to be placed thereon;
light projecting means comprising a light emitting element configured to emit light and collimating means for converting the light emitted from the light emitting element after convergence into parallel light toward a detection area; and
light receiving means for receiving the light passing through the detection area from the collimating means at a position located on a same plane as the placement table in a direction perpendicular to an optical axis of the light,
wherein the optical axis of the light emitted from the light projecting means is set such that the light is incident in a direction perpendicular to an incident surface of the light receiving means.

3. The photoelectric sensor according to claim 1 or 2,
wherein the light emitting element is a laser diode,
wherein the photoelectric sensor further comprises an optical fiber having a core diameter smaller than a light emitting area of the laser diode, and
wherein light emitted by the light projecting means via the optical fiber and received by the light receiving means is smaller than the light emitting area of the laser diode.

4. The photoelectric sensor according to claim 3, wherein the optical fiber is configured as a single-mode fiber.

5. The photoelectric sensor according to any one of claims 1 to 4, further comprising:
a display unit configured to display measured resin transmittance.

6. A method for measuring resin transmittance in laser resin welding, the method comprising:
a step of preparing the photoelectric sensor according to any one of claims 1 to 5;
a step of placing only a laser beam transmissive resin configured to allow a laser to pass therethrough, which is included in a plurality of resins to be laser-welded, on the incident surface of the light receiving means on a surface where the laser beam transmissive resin is to be welded; and
a step of measuring resin transmittance which is laser beam transmittance of the laser beam transmissive resin.

7. A laser resin welding method comprising:
a step of preparing the photoelectric sensor according to claim 5;
a step of placing a laser beam transmissive resin configured to allow a laser to pass therethrough, which is included in a plurality of resins to be laser-welded, on the placement table of the photoelectric sensor;
a step of measuring resin transmittance which is laser beam transmittance of the laser beam transmissive resin;
a step of setting a machining condition of a laser machining device based on the resin transmittance displayed on the display unit; and
a step of performing laser welding of the laser beam transmissive resin by the laser machining device under the set machining condition.

8. A laser machining device comprising:
the photoelectric sensor according to any one of claims 1 to 5; and
a laser machining device body configured to emit a laser having a wavelength that is the same as or approximated to a wavelength of a laser emitted by the photoelectric sensor.

9. A laser machining device comprising:
the photoelectric sensor according to any one of claims 1 to 5; and
a laser machining device body configured to receive data of laser beam resin transmittance measured by the photoelectric sensor and transmitted from the photoelectric sensor, and perform laser machining based on the data.
